(19) Europäisches Patentamt
European Patent Office
Office européen des brevets

(11) EP 2 987 514 B1

(12) EUROPEAN PATENT SPECIFICATION

(45) Date of publication and mention of the grant of the patent:
**21.12.2022 Bulletin 2022/51**

(21) Application number: **14784797.4**

(22) Date of filing: **14.02.2014**

(51) International Patent Classification (IPC):
***A61M 1/18*** *(2006.01)*

(52) Cooperative Patent Classification (CPC):
**A61M 1/16; B01D 67/0011; B01D 69/02; B01D 69/087; B01D 71/68**

(86) International application number:
**PCT/JP2014/053488**

(87) International publication number:
**WO 2014/171172 (23.10.2014 Gazette 2014/43)**

(54) **HOLLOW FIBER MEMBRANE FOR BLOOD TREATMENT**

HOHLFASERMEMBRAN ZUR BLUTBEHANDLUNG

MEMBRANE À FIBRES CREUSES POUR LE TRAITEMENT DU SANG

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.04.2013 JP 2013088352**

(43) Date of publication of application:
**24.02.2016 Bulletin 2016/08**

(73) Proprietor: **Asahi Kasei Medical Co., Ltd.
Tokyo 100-0006 (JP)**

(72) Inventors:
- **UDA, Miwako
Tokyo 101-8101 (JP)**
- **SEKINE, Kumiko
Tokyo 101-8101 (JP)**
- **KAWAKAMI, Junya
Tokyo 101-8101 (JP)**

(74) Representative: **dompatent von Kreisler Selting Werner -
Partnerschaft von Patent- und Rechtsanwälten mbB
Deichmannhaus am Dom
Bahnhofsvorplatz 1
50667 Köln (DE)**

(56) References cited:
**EP-A1- 0 749 775**
**EP-A2- 0 850 678**
**EP-A2- 0 923 955**
**WO-A1-2008/146775**
**WO-A1-2011/090197**
**WO-A1-2012/169529**
**WO-A1-2013/015046**
**JP-A- H0 966 225**
**JP-A- H07 178 166**
**JP-A- H10 235 171**
**JP-A- H10 244 000**
**JP-A- H11 178 919**
**JP-A- H11 347 117**
**JP-A- 2006 296 931**

- **MASATOMI SASAKI: "Development of vitamin E-modified polysulfone membrane dialyzers", JOURNAL OF ARTIFICIAL ORGANS ; THE OFFICIAL JOURNAL OF THE JAPANESE SOCIETY FOR ARTIFICIAL ORGANS, SPRINGER-VERLAG, TO, vol. 9, no. 1, 1 March 2006 (2006-03-01), pages 50-60, XP019374448, ISSN: 1619-0904, DOI: 10.1007/S10047-005-0318-8**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 987 514 B1

## Description

### Technical Field

The present invention relates to a hollow fiber membrane for blood treatment, particularly a polysulfone hollow fiber membrane for blood treatment, and a method for producing the hollow fiber membrane for blood treatment.

### Background Art

Conventionally, hollow fiber membrane type blood purifiers, such as hemodialyzers, hemofilters, or hemodiafilters, including hollow fiber membranes for blood treatment using polymers, such as cellulose, cellulose acetate, polysulfone, polyethersulfone, polymethylmethacrylate, or polyacrylonitrile, as separation materials have been widely used in blood purification therapies for kidney failure therapy and the like for the purpose of removing uremic toxins and waste products in the blood. These hollow fiber membrane type blood purifiers are excellent in terms of the decrease in the amount of extracorporeal circulating blood, the efficiency of removing uremic substances in the blood, productivity in module production, and the like; and a polysulfone hollow fiber membrane, which can combine high permeability and blood compatibility, is particularly excellent. The polysulfone hollow fiber membrane is preferable also because the membrane has high affinity to fat-soluble vitamins that are effective for reducing oxidative stress brought with the extracorporeal circulation of blood and enables the fat-soluble vitamins to be easily immobilized on the membrane surface (Patent Literatures 1 and 2).

In this field, various proposals have been made for obtaining blood purifiers having high molecular weight fractionation intended to remove β2 microglobulin (β2MG molecular weight: 11800) as a causative substance for dialysis amyloidosis and reduce the loss of albumin (molecular weight: 66000) as a useful protein. For example, Patent Literature 3 reports that for a hollow fiber membrane of an asymmetric structure having a compact layer at least on the inner surface side, its fractionating properties are improved by controlling the hollow inner diameter, the membrane thickness, the compact layer thickness, and the fracture elongation; however, the fractionating properties are not yet sufficient and it is uncertain whether the improvement can be applied to the polysulfone hollow fiber membrane because the membrane material is limited to a cellulose acetate polymer.

Patent Literature 4 reports that for a hollow fiber membrane mainly consisting of a polysulfone polymer and polyvinylpyrrolidone, its fractionating properties between α1 microglobulin (molecular weight: 33000) and albumin are improved by controlling the abundance ratio of the two polymers on the blood contact side and the non-contact side; however, according to an experiment by the present inventors, the fractionating properties between β2MG and albumin were not sufficient.

In addition, albumin itself has recently been pointed out to have the possibility of becoming a uremic toxin by the oxidation of albumin by oxidative stress or the adsorption of a uremic toxin by albumin. According to this theory, it is considered that a certain amount of albumin should be positively removed to promote the metabolism of albumin, as is done in vivo by the kidney. Further, a hollow fiber membrane is demanded, which has higher permeability in conjunction with improvement in the amount of (β2MG removal continuing to be demanded.

Disequilibrium syndrome has previously been known, which exhibits symptoms, such as headache, nausea, or vomiting, due to the osmotic pressure difference between the blood whose uremic toxin concentration is sharply decreased with blood purification therapy and the body tissue in which the uremic toxin concentration is easily maintainable. These do not immediately cause threat to life or serious physical impediment but are a distress for patients; a measure taken for symptom relief, for example, a change to a small membrane area blood purifier, results in reduced therapeutic effect, or the syndromes are a burden for healthcare professionals due to symptomatic treatment at the occurrence of the symptoms.

### Citation List

### Patent Literature

Patent Literature 1: Japanese Patent Application Laid-Open No. 07-178166
Patent Literature 2: Japanese Patent Application Laid-Open No. 2006-296931
Patent Literature 3: Japanese Patent Application Laid-Open No. 2011-212638
Patent Literature 4: Japanese Patent Application Laid-Open No. 2005-270631


Furthermore, EP0923955 discloses polysulfone hollow fiber membranes coated with vitamin E by filling the

lumen of said membrane with a solution comprising said vitamin in n-hexane followed by drying in nitrogen gas.

## Summary of Invention

### Technical Problem

Albumin is responsible for much of colloid osmotic pressure; however, albumin leakage in a level of becoming a problem did not occur in conventional hollow fiber membranes. According to an experiment by the present inventors, the protein permeation amount of a hollow fiber membrane whose permeability is increased by extending conventional technology is initially high, sharply decreases, and reaches a constant level about 1 hour later. For this reason, aiming at a certain amount of albumin leakage in single treatment will result in a drastic reduction in the albumin concentration immediately after the start of therapy and may increase the risk of disequilibrium syndrome as compared to before.

For this reason, a hollow fiber membrane for blood treatment is desirable, which has high solute permeability enabling a certain amount of albumin leakage to be achieved in blood treatment and further has a reduced amount of albumin leakage at the beginning of the blood treatment, i.e., continues to gently leak albumin over the entire period of the blood treatment, in addition to having improved fractionating properties between β2MG and albumin as conventionally required. However, in conventional technology, such a blood purifier has not been present.

Accordingly, an object of the present invention is to provide a hollow fiber membrane for blood treatment that can be used in a blood purifier having a high solute permeability enabling a certain amount of albumin leakage to be achieved in blood treatment, where a time-course change in albumin leakage can be reduced over the entire period of the blood treatment, and a method for producing the same.

### Solution to Problem

As a result of intensive studies for solving the above problems, the present inventors have found that a polysulfone hollow fiber membrane for blood treatment as defined in the appended claims, comprising a polysulfone resin, a hydrophilic polymer, and a fat-soluble substance, in which the surface of the membrane contains 10 to 300 mg of the fat-soluble substance per square meter; the content of the fat-soluble substance present in the surface of the membrane is 40 to 95% by mass when the content of the fat-soluble substance present in the entire membrane is assumed as 100% by mass; and further the clearance rate of β2MG converted to that for 1.5 $m^2$ for a blood purifier assembled using the membrane is 65 mL/min or more, solves the above problems, thereby achieving the present invention.

The present polysulfone hollow fiber membranes are as defined in the appended claims.

### Advantageous Effects of Invention

The present invention can provide a hollow fiber membrane for blood treatment which has high solute permeability enabling a certain amount of albumin leakage to be achieved in blood treatment and has a reduced amount of albumin leakage at the beginning of the blood treatment, i.e., continues to gently leak albumin over the entire period of the blood treatment, in addition to having improved fractionating properties between β2MG and albumin, and a method for producing the hollow fiber membrane for blood treatment.

### Brief Description of Drawings

[Figure 1] Figure 1 is a schematic diagram showing "single circuit" in a blood purifier having a membrane area of 1.5 $m^2$.

[Figure 2] Figure 2 is a schematic diagram showing "closed circuit" in a blood purifier having a membrane area of 1.5 $m^2$.

### Description of Embodiment

The following explanations are all described by assuming a case where blood flows through the lumen of a hollow fiber membrane as in a blood dialyzer; however, the words meaning the inside and outside of the hollow fiber membrane shall be reversely read in a case where blood does not flow through the lumen of the hollow fiber membrane, i.e., a case where blood flows through the outside of the hollow fiber membrane.

<Polysulfone Hollow Fiber Membrane for Blood Treatment>

The hollow fiber membrane for blood treatment according to the present embodiment (hereinafter, sometimes simply referred to as "hollow fiber membrane") comprises a polysulfone resin, a hydrophilic polymer, and a fat-soluble substance, wherein the hollow fiber membrane contains 10 to 300 mg of the fat-soluble substance per square meter in the membrane surface; the content of the fat-soluble substance present in the surface of the hollow fiber membrane is 40 to 95% by mass when the content of the fat-soluble substance present in the entire membrane is assumed as 100% by mass; and the clearance rate of β2MG converted to that for 1.5 $m^2$ for a blood purifier constructed using the hollow fiber membrane is 65 mL/min or more.

The hollow fiber membrane is a hollow fiber membrane for blood treatment used in a blood purifier. The "blood purifier" is a device for purifying blood, used for extracorporeal blood circulation therapy, such as a hemodialyzer, a hemodiafilter, a hemofilter, or a continuous hemo(dia)filter.

Forms, such as the inner diameter, the thickness, and the length, can be arbitrarily adjusted. For example, the inner diameter may be selected from the range of 100 to 300 μm; the thickness, 10 to 100 μm; and the length, 10 to 40 cm. In order for the hollow fiber membrane to combine high molecular weight fractionating properties and high water permeability, it is preferable that the membrane is a so-called asymmetric membrane, haying a thin compact layer (active separation layer) and a porous layer responsible for hollow fiber strength (support layer). The hollow fiber membrane for blood treatment according to the present embodiment can have a structure similar to those of conventionally known hollow fiber membranes for blood treatment as described, for example, in Patent Literatures 1 and 2; however, these conventional hollow fiber membranes do not aim at having high permeability and are not intended for fractionating properties between β2MG and albumin becoming manifest in a high permeability blood purifier, or the relief of disequilibrium syndrome shown in the present invention. On the other hand, the present invention is intended for a hollow fiber membrane used in a high-performance blood purifier, and the clearance of β2MG converted to that for 1.5 $m^2$ for a blood purifier obtained by assembling the hollow fiber membrane of the present embodiment is 65 mL/min or more, preferably 70 mL/min or more. The β2MG clearance converted to that for 1.5 $m^2$ defined in the present invention is measured and calculated by the following method.

<Measurement of β2MG Clearance>

The β2MG clearance is measured in accordance with a measurement method defined in Takeshi Satoh, the Journal of the Japanese Society for Dialysis Therapy (1996) vol. 29(8): 1231-1245. A specific example will be presented below.

A test fluid used on the blood side is bovine plasma obtained by subjecting anticoagulated bovine whole blood to plasma separation. The anticoagulant may be a citrate anticoagulant or may be heparin. A total plasma protein level is adjusted to 6.5 ± 0.5 g/dL, and a commercially-available β2MG reagent is added to make its concentration at 0.01 to 4 mg/L. As a dialysis fluid, a commercially available dialysis fluid having a pH of around 7.4 or an equivalent buffer solution prepared may be used. Test fluids on the blood and dialysis fluid sides are both maintained at 37 ± 1°C. A blood purifier having a membrane area of 1.5 $m^2$ is set in the "single circuit" shown in Figure 1; the inlet flow rate on the blood side (QBi) of the blood purifier is adjusted to 200 mL/min; the flow rate on the blood outlet side (QBo), to 185 mL/min; and the dialysis fluid inlet flow rate (QDi), to 500 mL/min. After stationary waiting is carried out until the outlet solute concentration on the blood side, CBo, is stabilized, fluids are sampled from the outlets of the blood supply fluid and the test fluid on the blood side and the outlet of the test fluid on the dialysis fluid side, and the respective β2MG concentrations, CBi, CBo, and CDo, are measured. The β2MG concentration may be measured using a commercially available apparatus, or the measurement may be requested to a laboratory test company. The clearance (CL) is calculated by expression (1).
[Expression 1]

$$CL = (200 \times CBi - 185 \times CBo) / CBi \quad \cdots (1)$$

For a blood purifier having a membrane area other than 1.5 $m^2$, the measured value is converted to a value for 1.5 $m^2$ in reference to the method described in Michio Mineshima, "Ketsueki Johkaki - Seinohhyohkano Kiso (Blood Purifier - Basis for Performance Evaluation)", published by Nippon Medical Center (2002), The 1st Edition, 1st Issue. First, the β2MG clearance is measured by the above method except for resetting QBo by expression (2).
[Expression 2]

$$QBo = 200 - 10 \times A \quad (A: \text{membrane area, } m^2) \quad \cdots (2)$$

Next, the overall mass transfer coefficient (KO) is calculated by expression (3).
[Expression 3]

$$KO=\frac{QBi}{A\,(1-QBi/QDi)}\times \ln\left(\frac{1-CL/QDi}{1-CL/QBi}\right)\quad\cdots(3)$$

, where QBi = 200 mL/min; QDi = 500 mL/min; A = the membrane area ($m^2$) of test module; and CL = a clearance measured value (mL/min).

In addition, the clearance converted to that for 1.5 $m^2$ is calculated by expression (4).
[Expression 4]

$$CL=QBi\times\frac{1-\exp\left[K0\times A\left(1/QBi-1/QDi\right)\right]}{QBi/QDi-\exp\left[K0\times A\left(1/QBi-1/QDi\right)\right]}\cdots(4)$$

, where QBi = 200 mL/min; QDi = 500 mL/min; A = 1.5 $m^2$; and KO = the value determined by expression (3).

<Polysulfone Resin>

The polysulfone resin (hereinafter, referred to as "PSf" in some cases) of the present embodiment is a generic term applied to high molecular compounds having sulfone bonds, and is not particularly limited. Examples thereof include polysulfone polymers having repeat units represented by the following chemical formulas (1) to (5). In this respect, n is a polymerization degree and may be any value.

[Formula 1]

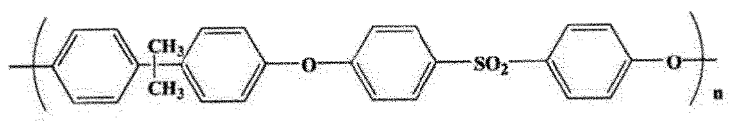

(1)

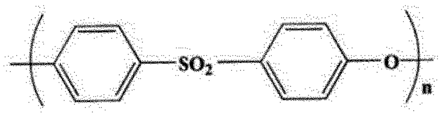

(2)

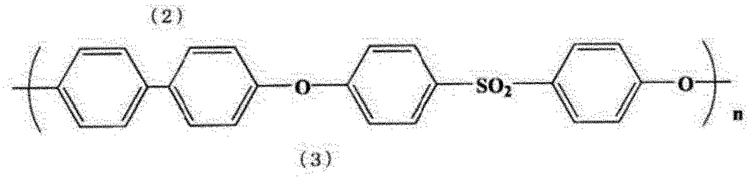

(3)

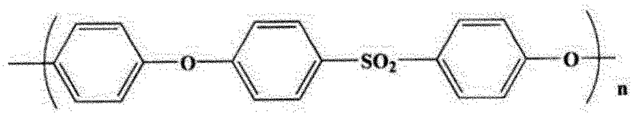

(4)

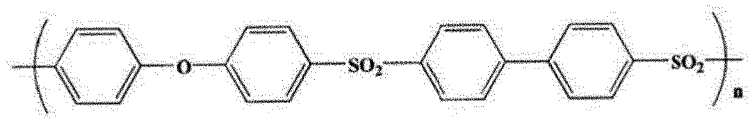

(5)

PSf of the chemical formula (1) is sold under the trade name "Udel" by Solvay Advanced Polymers Co., Ltd. and under the trade name "Ultrason" by BASF and a plurality of types are present depending on the polymerization degree; however, it is not particularly limited.

Examples of PSf of the chemical formula (2) include SUMIKAEXCEL PES (product name, from Sumitomo Chemical Co., Ltd.) and Ultrason (product name, from BASF). These are preferable because of being widely commercially available and capable of being easily obtained, and it is preferable for their reduced viscosity as measured in a 1 (w/v)% dimethylformamide (DMF) solution to be in the range of 0.30 to 0.60, more preferably in the range of 0.36 to 0.50, in view of ease of handling and ready availability.

<Hydrophilic Polymer>

Examples of the hydrophilic polymer of the present embodiment include polyvinylpyrrolidone (hereinafter, sometimes referred to as "PVP"), polyethylene glycol, polyvinyl alcohol, and polypropylene glycol; PVP is preferably used in view of fiber spinning stability and affinity to PSf.

For PVP, a plurality of types are present depending on the polymerization degree; for example, PVPs having different molecular weights, such as K-15, 30, and 90, are present under the trade name "Plasdone" from ISP Co., Ltd., and all of them can be used.

<Fat-soluble Substance>

The fat-soluble substance of the present disclosure generally refers to a substance soluble slightly in water and soluble in alcohol and fat and oil, and a natural or synthetic product having low toxicity may be used. Specific examples thereof include cholesterol; vegetable oils, such as castor oil, lemon oil, and shea butter; animal oils, such as fish oil; fatty acids, such as sucrose fatty acid ester and polyglyceryl fatty acid ester; isoprenoids; and hydrocarbons having a large number of carbons. Fat-soluble vitamins presently used, are selected from vitamin A, vitamin D, vitamin E, vitamin K, and ubiquinone. Among these, vitamin E is preferable in view of not causing any disorder even when excessively ingested.

The vitamin E may be α-tocopherol, α-tocopherol acetate, α-tocopherol nicotinate, β-tocopherol, γ-tocopherol, δ-tocopherol, or a mixture thereof. Among others, α-tocopherol is preferable because it is excellent in various physiological actions, such as an in vivo antioxidant action, a biomembrane stabilizing action, and a platelet aggregation inhibition action and highly effective in reducing the above oxidative stress.

The above fat-soluble substance may be one obtained by properly chemically modifying any of the above-exemplified compounds for the adjustment of antioxidative potency or affinity to a membrane base material, the improvement of stability, or other purposes.

As a result of intensive studies, the present inventors have found that a fat-soluble can be contained in a polysulfone hollow fiber membrane for blood treatment, constituting a blood purifier having a β2MG clearance rate converted to that for 1.5 $m^2$ of 65 mL/min or more to improve its fractionating properties and cause the gentle leak of albumin.

The reason for this is not apparent; however, it can be inferred as follows. Albumin is known to have a hydrophobic surface with respect to the molecule and adsorb to a hydrophobic surface. For this reason, it is presumed to also firmly adsorb to the surface of the pores of the separation active layer determining molecular fractionation of the present embodiment, in which the membrane surface is modified with a hydrophobic fat-soluble substance. On the other hand, albumin has negative charges, and the negative charge membrane is known to be capable of reducing the leak of albumin. The albumin strongly adsorbed to the membrane surface exerts an electrostatic repulsion effect on albumin getting close thereto afterwards, while not affecting the permeability of uremic substances, such as β2MG, having no charge. A similar phenomenon occurs in some of common PSf hollow fiber membranes, but it is probable that the effect prominently appears in the hollow fiber membrane of the present embodiment, whose surface is modified with a fat-soluble substance, and has been identified as an improvement in the fractionating properties between β2MG and albumin.

The firm adsorption of albumin to the pore surface of the separation active layer translates into the narrowing of the substantial inner diameter of the pores; thus, the pore diameter distribution of the hollow fiber membrane of the present embodiment is set to be that of larger pore diameters than those of conventional hollow fiber membranes. In a PSf hollow fiber membrane for blood purification, the pore structure is formed by the phase separation induced by adding a non-solvent to a PSf solvent. In a mixed solution of the PSf solvent and the non-solvent, the concentration of the solvent is 50 to 60% as an example. To obtain the structure of rather large pores necessary in the present embodiment, it is necessary to drive phase separation; however, the proportion of coarse pores is stochastically predicted to be increased. The above electrostatic repulsion is inversely proportional to the square of distance; thus, its effect is not obtained for the coarse pores. However, it is probable that these coarse pores present in a small but certain number continuously leak albumin, i.e., gently leak albumin.

<Amount of Fat-soluble Substance Present in Membrane Surface>

In the hollow fiber membrane of the present embodiment, the amount of the fat-soluble substance defined in the claims present in the membrane surface is 10 to 300 mg per square meter of membrane area, and further the content of the fat-soluble substance present in the surface of the hollow fiber membrane is 40 to 95% by mass when the content of the fat-soluble substance in the entire hollow fiber membrane is assumed as 100% by mass. The "content of the fat-soluble substance present in the surface of the hollow fiber membrane" refers to the content of the fat-soluble substance attached, adsorbed, or coated on the surface of the hollow fiber membrane, and the content of the fat-soluble substance present in the surface can be quantified, for example, based on the content of the fat-soluble substance extracted with a solvent without destructing or dissolving the hollow fiber membrane.

The amount of the fat-soluble substance present in the surface of the hollow fiber membrane is in the range of 10 $mg/m^2$ to 300 $mg/m^2$ (both inclusive) in terms of that per the membrane area of the hollow fiber membrane. It is preferably 50 to 250 $mg/m^2$, more preferably 100 to 200 $mg/m^2$. For the purpose of the present invention, the membrane area of the hollow fiber membrane refers to the effective total surface area of the hollow fiber membrane responsible for filtration and dialysis and is specifically the interior surface area of the hollow fiber membrane, and is calculated from the product of the average inner diameter (diameter) of the hollow fiber membrane, the circular constant, the number of the fibers, and the effective length thereof.

The content of the fat-soluble substance present in the membrane surface of 10 $mg/m^2$ or more can prevent the coating unevenness of the fat-soluble substance and provides the well-reproducible control of the albumin permeability. It can also exert an excellent antioxidant ability. On the other hand, the content exceeding 300 $mg/m^2$ cannot achieve high permeability as a feature of the present invention because the active separation layer in the hollow fiber membrane may be completely blocked by the fat-soluble substance.

An example of a method for measuring the content of the fat-soluble substance present in the hollow fiber membrane surface will be explained. First, the hollow fiber membrane type blood purifier is taken apart, and the hollow fiber membrane is collected, washed with water, and then subjected to drying treatment. Subsequently, a surfactant dissolving the fat-soluble substance, for example, a 1% by mass polyethylene glycol-t-octylphenyl ether aqueous solution, is added to the accurately weighed dried hollow fiber membrane, followed by stirring and extraction. The area of the extracted hollow fiber membrane is calculated from weight. The quantification operation is performed, for example, by the following liquid chromatography method, and the concentration of the fat-soluble substance in the extract is calculated using a calibration curve obtained from the peak area of a standard solution of the fat-soluble substance. The amount ($mg/m^2$) of the fat-soluble substance present in the hollow fiber membrane surface can be determined from the resultant concentration and the membrane area of the extracted hollow fiber membrane by assuming an extraction efficiency of 100%.

[One Example of Quantification Method Using Liquid Chromatography Method]

A high-performance liquid chromatography apparatus (pump: JASCO PU-1580, detector: Shimadzu RID-6A, autoinjector: Shimadzu SIL-6B, data processing: Tosoh GPC-8020, and Column Oven: GL Sciences556) is equipped with a column (ODP-506E packed column for HPLC from Shodex Asahipak), through which methanol for high-performance liquid chromatography as a mobile phase is then passed at a column temperature of 40°C, for example, at a flow rate of 1 mL/min, followed by determining the fat-soluble substance concentration from the absorption peak area at a wavelength of 295 nm with a UV detector.

<Proportion of Fat-soluble Substance Present in Hollow Fiber Membrane Surface>

The content of the fat-soluble substance present in the hollow fiber membrane surface in the hollow fiber membrane of the present embodiment is 40 to 95% of the content of the fat-soluble substance present in the entire hollow fiber membrane. It is preferably 45 to 90%, more preferably 50 to 80%.

An example of a method for measuring the content of the fat-soluble substance in the entire hollow fiber membrane will be explained. First, the hollow fiber membrane type blood purifier is taken apart, and the hollow fiber membrane is collected, washed with water, and then subjected to drying treatment. Subsequently, a solvent for PSf, for example, N-methyl-2-pyrrolidone, is added to the accurately weighed dried hollow fiber membrane, followed by stirring and dissolution. The membrane area of the dissolved hollow fiber membrane is calculated from weight. The quantification operation is performed, for example, by the liquid chromatography method, and the concentration of the fat-soluble substance in the solution is calculated using a calibration curve obtained from the peak area of a standard solution of the fat-soluble substance. The mass ($mg/m^2$) of the fat-soluble substance contained in the hollow fiber membrane can be determined from the resultant concentration and the membrane area of the dissolved hollow fiber membrane.

The proportion of the fat-soluble substance present in the hollow fiber membrane surface can be calculated by

expression (5).
[Expression 5]

$$\text{Proportion of fat-soluble substance present in hollow fiber membrane surface} = \text{mass of fat-soluble substance present in membrane surface} / \text{mass of fat-soluble substance contained in membrane} \times 100\ (\%) \quad \cdots (5)$$

When the content of the fat-soluble substance present in the hollow fiber membrane surface is 40% or less of the content of the fat-soluble substance in the entire hollow fiber membrane, it is difficult to improve its fractionating properties and to cause the gentle leak of albumin. This is due to a decrease in the amount of the fat-soluble substance immobilized on the hollow fiber membrane surface. Increasing the content of the fat-soluble substance in the entire membrane to avoid this cannot be adopted because there is a risk that permeability is changed by the movement of a large amount of the fat-soluble substance in the entire membrane toward the membrane surface due to thermal history and a lapse of time during the storage of the blood purifier. On the other hand, when the value exceeds 95%, there is an increasing risk of causing a change in permeability conversely due to the movement of the fat-soluble substance from the membrane surface toward the membrane base material. The high permeabilities of the hollow fiber membrane of the present embodiment and the blood purifier using the membrane reflect the high balance between the risk of excessively leaking albumin and a reduced therapeutic effect, conversely, due to insufficiently leaking albumin, and the risk of variation in permeability should be avoided whenever possible.

<Albumin Leakage Amount>

When blood dialysis is taken for example as blood treatment, it is desirable that the albumin leakage amount in the whole single therapy be 1.0 to 10 g/session, preferably 1.2 to 9.0 g/session, more preferably 1.5 to 8.0 g/session. With the leakage amount of less 1.0 g/session, the effect of removing albumin which has become uremic toxin and promoting metabolism is difficult to be exhibited, and with the leakage amount of more than 10 g/session, the continuation of therapy causes the risk of developing hypalbuminosis. As used herein, the session means the therapy time for single blood treatment, and is, for example, ordinarily 240 minutes (4 hours) for blood dialysis. According to an experiment by the present inventors, if the filtration flow rate per membrane area is made constant, the change of the membrane area in the blood purifier does not cause a large difference in the measured value of the albumin leakage amount. Thus, the membrane area in the blood purifier used for measurement is not particularly limited; however, it is preferable for the area to be 1.5 $m^2$ as the predominant membrane area or close thereto. The albumin leakage amount in the whole single therapy in the case of using the blood purifier of the present embodiment can be estimated by a 240-minute (1 session) simulation therapy experiment using a measurement method as described below.

<Albumin Leakage Amount Measurement>

Taking dialysis therapy as a blood treatment therapy for example, the method for measuring the albumin leakage amount will be described below.

Bovine blood whose coagulation is reduced by adding citric acid or heparin sodium is used and diluted with bovine plasma or physiological saline to prepare one having a hematocrit of 35 $\pm$ 2% and a total protein concentration of 6.5 $\pm$ 0.5 g/dL. A commercially available dialysis fluid is fed through the blood flow channel of the blood purifier at 500 mL/min by one pass while circulating the blood through the blood flow channel of the blood purifier at 200 mL/min, and dialysis is performed while conducting ultrafiltration at 6 ml/min/$m^2$. A commercially available dialyzer (for example, DBG-03 from Nikkiso Co., Ltd.) is used for feeding the dialysis fluid. To prevent hemolysis, the blood purifier is sufficiently purged with physiological saline in advance. The feeding of the dialysis fluid and the ultrafiltration are started simultaneously with the start of bovine blood circulation, and the sampling of a ultrafiltrated fluid is started. When the sampling of only the ultrafiltrated fluid is difficult, all dialysis waste fluid may be collected. The ultrafiltrated fluid or the dialysis waste fluid is sampled by being integrated for predetermined periods of time (for example, integrated for 0 to 5 minutes, next for 5 minutes to 1 hour, and then for 1 to 4 hours, or repeating 5-minute integration for 4 hours) to make test fluids. Each of the test fluids is well mixed, and the concentration of albumin in the test fluid is quantified, for example, on an autoanalyzer, Biolis24i (from Tokyo Boheki Medical System), using Iatro U-ALB(TIA) reagent (from Mitsubishi Chemical Medience Co., Ltd.). The leakage amount of albumin per 1 session (240 minutes) (4-hour value) is calculated using

expression (6) or expression (7).
[Expression 6]

$$\text{Albumin leakage amount (g)} = \text{albumin concentration in test fluid} \times (240 \times 500 + \text{ultrafiltrated fluid amount}) \quad \cdots (6)$$

[Expression 7]

$$\text{Albumin leakage amount (g)} = \text{albumin concentration in test fluid} \times \text{dialysis waste fluid amount} \quad \cdots (7)$$

<Proportion of 5-Minute Integrated Value of Albumin Leakage Amount with respect to 1-Hour Integrated Value of Albumin Leakage Amount>

The proportion of the albumin leakage amount from the start of blood treatment until 5 minutes thereafter (referred to as "5-minute integrated value of the albumin leakage amount" in some cases) with respect to the albumin leakage amount from the start of blood treatment until 1 hour thereafter (referred to as "1-hour integrated value of the albumin leakage amount" in some cases) may be 50% or less, is preferably 45% or less, more preferably 40% or less. A proportion of the amounts of more than 50% sharply decreases the albumin concentration immediately after the start of dialysis and increases the risk of inducing disequilibrium syndrome. The proportion of the 5-minute integrated value of the albumin leakage amount with respect to the 1-hour integrated value of the albumin leakage amount when the blood purifier of the present embodiment is used may be determined by measuring the integrated value for 5 minutes after the start of blood treatment and the integrated value for 1 hour after the start of blood treatment, respectively, by the above albumin leakage amount measurement method and calculating their ratio.

<Proportion of 5-Minute Integrated Value of Albumin Leakage Amount with respect to Total Integrated Value of Albumin Leakage Amount>

It is desirable that the proportion of the 5-minute integrated value of the albumin leakage amount with respect to the albumin leakage amount over the total period of time of blood treatment (referred to as "total integrated value of albumin leakage amount" in some cases) be 30% or less, preferably 25% or less, more preferably 20% or less. A proportion of the values of more than 30% sharply decreases the albumin concentration immediately after the start of dialysis and increases the risk of inducing disequilibrium syndrome. In the present embodiment, the total period of therapy (1 session) is set at 240 minutes (4 hours), and the total integrated value of albumin leakage amount is set at the 4-hour integrated value of the albumin leakage amount. The proportion of the 5-minute integrated value of the albumin leakage amount with respect to the total integrated value of albumin leakage amount (or the 4-hour integrated value of the albumin leakage amount) when the blood purifier of the present embodiment is used may be determined by measuring the integrated value for 5 minutes after the start of blood treatment and the integrated value for 4 hours as the total treatment period, respectively, and calculating their ratio.

<Ketone and/or Alcohol Having 1 to 4 Carbons (Component A)>

The performance of a hollow fiber membrane has conventionally decreased in a step of adding a fat-soluble substance. As described above, the present inventors assume that the effect of gently leaking albumin according to the present application stems from a small number of coarse pores present in the separation active layer of the membrane. Thus, the occurrence of a change in the distribution of membrane pore diameters in a step of adding a fat-soluble substance does not provides a desired level of albumin leakage, resulting in a risk of excessive or insufficient leakage. The decreased performance of the hollow fiber membrane in the step of adding a fat-soluble substance is probably due to that: the fat-soluble substance and the base material resin of the hollow fiber membrane have, though low, compatibility with each other; the structure-retaining force of the base material resin is decreased; and the pore diameter distribution is changed. It is probable that if the base material resin of a hollow fiber membrane had no compatibility with a fat-soluble substance, the fat-soluble substance would only adhere as an oil layer to the surface of the membrane base material and not migrate into the membrane base material PSf and no change in the pore diameter distribution would occur.
The present inventors have succeeded in decreasing the compatibility between a fat-soluble substance and a

hydrophobic resin by adding a ketone and/or an alcohol having 1 to 4 carbons (hereinafter, referred to as "component A" in some cases) to the fat-soluble substance, and have succeeded in reducing migration into the hydrophobic resin as the base material of a hollow fiber membrane and a subsequent decrease in the structure-retaining force. This decreases the reduction of a change in performance, and eventually decreases variation in the performance of a resultant blood purifier. Thus, the hollow fiber membrane of the present embodiment is a hollow fiber membrane composed of a hydrophobic resin, a hydrophilic resin, and a fat-soluble substance, and preferably further contains 2 to 3000 μg of the component A per gram of the hollow fiber membrane. The hollow fiber membrane of the present embodiment contains the component A to reduce variation in the reduction of the albumin leakage amount, providing a more stable effect of reducing the development of disequilibrium syndrome. The variation can be quantified using CV value (= (the standard deviation of measured values) ÷ (the average value of the measured values) for the measurement of the albumin leakage amount; however, it is preferable for the CV value to be less than 50%.

The component A, i.e., a ketone and/or an alcohol having 1 to 4 carbons, is at least one compound selected from the group consisting of ketones each having 1, 2, 3, or 4 carbons and alcohols each having 1, 2, 3, or 4 carbons. Examples of the component A include, but not limited to, acetone, methyl ethyl ketone, methanol, ethanol, propyl alcohol, isopropyl alcohol, ethylene glycol, glycerin, and butyl alcohol; these compounds may be used alone or in any combination thereof. Among these, acetone, ethanol, and isopropyl alcohol are preferably used. Use of a compound other than the component A, for example, an ether compound or tetrahydrofuran, may cause a change in the structure of the hydrophobic resin due to swelling or dissolution and thereby markedly decrease performance.

According to previous research by the present inventors, the effect of reducing a decrease in performance was noted only for a polyether sulfone represented by the above chemical formula (2) as PSf when attention is directed to the water permeation amount for the decrease in the performance of a hollow fiber membrane in a step of adding a fat-soluble substance. In subsequent research, an effect for the stability of coarse pores affecting albumin leakage mainly intended by the present applied invention became manifest for even PSf other than the polyether sulfone.

The method for causing the hollow fiber membrane to contain the component A will hereinafter be described.

The abundance of the component A contained in the hollow fiber membrane is is from 2 to 3000 μg, more preferably 10 to 2000 μg, still more preferably 25 to 1500 μg per gram of the hollow fiber membrane. The abundance of 2 μg/g or more can sufficiently decrease the compatibility between the fat-soluble substance and the hydrophobic resin, can secure the structure-retaining force of the hollow fiber membrane, and can prevent a decrease in membrane performance. Less than 2 μg/g markedly varies performance depending on the degree of abundance and has the possibility of producing variation in the albumin leakage amount. The abundance of 3000 μg or less can prevent the peeling of the fat-soluble substance during clinical implementation or the like and can prevent elution into blood, and a decrease or change in antioxidant performance when the fat-soluble substance is a fat-soluble antioxidant.

The content of the component A contained in the hollow fiber membrane per gram of the hollow fiber membrane can be measured by the following method.

[Case of Blood Purifier Filled with Water]

A blood purifier is disassembled; the hollow fiber membrane is separated; and water is removed therefrom by using a commercially available centrifuge under conditions of 500 rpm and 10 minutes or the respective larger values. A part or all of the hollow fiber membrane from which water has been removed is measured for mass (called "w1 (g)"). After measurement, the membrane is dissolved by adding, for example, a 100-fold mass of N-methyl-2-pyrrolidone (called "w2 (g)") and measured for the concentration x (w/w%) of the component A, for example, by gas chromatography. The content of the component A per gram of the hollow fiber membrane ("D (g/g of the hollow fiber membrane)") is calculated by substituting the values w1 and x into the following expressions, (8) to (9).

[Expression 8]

$$(\text{Mass of component A in solution}) = x \times (w1 + w2) \div 100 \quad \cdots (8)$$

[Expression 9]

$$D = (\text{Mass of component A in solution}) \div w1 \quad \cdots (9)$$

[Case of Dry Blood Purifier]

A hollow fiber membrane type blood purifier is disassembled, and the hollow fiber membrane bundle is separated. The bundle is dried to constant weight in a desiccator using calcium chloride or the like as a drying agent and weighed

for mass ("w3 (g)"), and N-methyl-2-pyrrolidone ("w4 (g)") is then added thereto, which is then stirred and dissolved, followed by calculating the concentration y (w/w%) of the component A by the above method. Subsequently, the content of the component A per gram of the hollow fiber membrane ("D (g/g of the hollow fiber membrane)") is calculated by substituting w3 and y into the following expression (10).

[Expression 10]

$$D = y \times (w3 + w4) \div w3 \div 100 \quad \cdot\cdot\cdot (10)$$

<Method for Producing Hollow Fiber Membrane>

The hollow fiber membrane of the present embodiment can be produced by using a well-known dry-wet membrane formation technique. The method has a spinning step of obtaining a membrane intermediate containing, for example, a polysulfone resin and a hydrophilic polymer by using dry-wet spinning method and an immobilization step of immobilizing a fat-soluble substance on the membrane intermediate; in the spinning step, a hollow-forming agent as a mixed solution of a solvent and a non-solvent for the polysulfone resin, in which the concentration of the solvent is 50 to 60%, may be used.

First, PSf and the hydrophilic polymer are dissolved in a common solvent to prepare a spinning stock solution. The common solvent is not particularly limited; however, examples thereof include solvents, such as dimethylacetamide (hereinafter, referred to as DMAC), dimethyl sulfoxide, N-methyl-2-pyrrolidone, dimethylformamide, sulfolan, and dioxane, or a mixed solvent of 2 or more of the above solvents. The pore structure is formed by phase separation induced by adding a non-solvent to a solvent for PSf. The added non-solvent is a mixed solution of a solvent and a non-solvent for PSf, in which the concentration of the solvent is 50 to 60%. To control the pore diameter of a desired hollow fiber membrane, an additive such as water may be added to the spinning stock solution.

The PSf concentration in the spinning stock solution is not particularly limited provided that it is in such a concentration range that a membrane can be formed and the resultant membrane has performance as a permeable membrane; preferred is 5 to 35% by mass, more preferably 10 to 30% by mass. To achieve high water-permeating performance, it is preferable for the PSf concentration to be lower, more preferably 10 to 25% by mass.

With regard to the concentration of the hydrophilic polymer in the spinning stock solution based on PSf, it is preferable for the mixing proportion of the hydrophilic polymer based on 100% by mass of PSf to be 27% by mass or less; the proportion is more preferably adjusted to 18 to 27% by mass, still more preferably 20 to 27% by mass.

A mixing proportion of the hydrophilic polymer based on PSf of more than 27% by mass tends to increase the elution amount of the hydrophilic polymer; less than 18% by mass is undesirable because it decreases the hydrophilic polymer concentration in the surface of the hollow fiber membrane and becomes a cause of inducing leukopenia symptoms in which the leukocyte concentration in the blood of a patient rapidly decreases.

In a step of producing the hollow fiber membrane, a tube-in-orifice type spinneret is used to extrude the spinning stock solution through the orifice of the spinneret simultaneously with a hollow inner solution for coagulating the spinning stock solution through the tube into the air.

The hollow inner solution may be water or a water-based coagulation fluid; its composition and the like may be determined depending on the permeability of a desired hollow fiber membrane. Commonly, a mixed solution of the solvent used in the spinning stock solution and water is preferably used. To obtain the hollow fiber membrane having high solute permeability according to the present embodiment, it is necessary for the solvent to have a high concentration; when the polysulfone represented by the above chemical formula (1) is used as PSf, a 50 to 70% by mass, preferably 50 to 65% by mass, aqueous solution thereof is necessary. In addition, the hydrophilic polymer can also be added to 0 to 2% by mass to the hollow inner solution to adjust the abundance of the hydrophilic polymer in the hollow fiber membrane inner surface.

The spinning stock solution extruded together with the hollow inner solution through the spinneret is run through a free-running section, introduced and immersed into a coagulating bath mainly containing water, installed on the lower portion of the spinneret for the completion of coagulation, rolled up on a hollow fiber membrane winder in a wet state after a washing step and the like to provide a hollow fiber membrane bundle, and then subjected to drying treatment. Alternatively, after the washing step, drying may be carried out in a drier to provide the hollow fiber membrane bundle.

By the above method, the mass of the hydrophilic polymer present in the inner surface of the hollow fiber membrane is made 25% by mass to 40% by mass (both inclusive) based on the total mass of the PSf and the hydrophilic polymer to provide a hollow fiber membrane excellent in antithrombogenicity and biocompatibility, to whose surface the adhesion of proteins, platelets, and the like is mild.

[Method for Producing Hollow Fiber Membrane Type Blood Purifier]

Preferred examples of a method for producing the hollow fiber membrane type blood purifier of the present embodiment include a method that involves first producing a hollow fiber membrane as described above and then producing a module using the hollow fiber membrane.

Specifically, the above hollow fiber membranes bundle is inserted into a tubed container having port openings for a predetermined fluid as a fluid being treated; a potting agent such as polyurethane is injected into both ends of the bundle to form potting layers to seal both ends; the hardened extra potting agent is then cutting-removed to open the end faces; and headers having port openings for the fluid are attached.

Subsequently, the fat-soluble substance is immobilized as will hereinafter be described, and further sterilization treatment is applied as will hereinafter be described. The immobilization of the fat-soluble substance may be carried out independently or simultaneously with the formation of the hollow fiber membrane as will hereinafter be described. In addition, the immobilization can also be performed at the stage of the hollow fiber membrane before modularization.

<Step of Immobilizing Fat-soluble Substance on Hollow Fiber Membrane>

The step of immobilizing the fat-soluble substance on the hollow fiber membrane can be basically carried out using any of well-known methods. Among others, a coating method is excellent in that it enables the realization of the production of fat-soluble substance-immobilized membranes having various permeabilities by using existing equipment and a product lineup.

The immobilization method of a fat-soluble substance on a hollow fiber membrane can be specifically performed by any of various methods, including a method that involves adding a fat-soluble substance to a membrane formation stock solution in forming the hollow fiber membrane to cause the entire hollow fiber membrane to contain the fat-soluble substance, a method that involves adding a fat-soluble substance and a surfactant to a hollow inner solution to cause the inner surface of the hollow fiber membrane to contain the fat-soluble substance (for example, Japanese Patent No. 4038583 and International Publication No. WO 98/52683) and a method that involves flowing a fat-soluble substance solution consisting of the fat-soluble substance and a solvent for the fat-soluble substance into the hollow portion of the hollow fiber membrane after assembling a hollow fiber membrane type blood purifier to cause the fat-soluble substance to adhere to the inner surface of the hollow fiber membrane (for example, Japanese Patent Application Laid-Open No. 2006-296931); however, any other method may also be used.

The hollow fiber membrane of the present embodiment has a fat-soluble substance content of 10 mg to 300 mg per square meter of the hollow fiber membrane; examples of a method for control in the range include the following method.

Specifically, when the fat-soluble substance is added to the membrane formation stock solution for the hollow fiber membrane, preferred examples of such a method include a method that involves adjusting the addition concentration to 0.4% by mass to 2% by mass, preferably 0.8% by mass to 1.5% by mass.

The addition concentration of 0.4% by mass or more can sufficiently secure the surface exposure amount of the fat-soluble substance and provide a favorable antioxidant ability. On the other hand, 2% by mass or less can practically sufficiently secure the strength of the hollow fiber membrane and prevent a risk of the breakage of the membrane.

The formed hollow fiber membrane can be further subjected to heat treatment at a temperature in the range of 100°C to 180°C, preferably 110 to 180°C, in a dry state to cause a larger amount of the fat-soluble substance to be localized on the surface of even a hollow fiber membrane having the same content of the fat-soluble substance.

The heating temperature of 100°C or more can exert the effect of localizing the fat-soluble substance; while the temperature of 180°C or less can prevent the softening of the hollow fiber membrane and a change in permeability.

In the case of addition to the hollow inner solution, it is preferable that the concentration of the fat-soluble substance be adjusted in the range of 0.01 to 10% by mass, preferably 0.1 to 5% by mass and an additive capable of making the fat-soluble substance soluble in the hollow inner aqueous solution, such as a surfactant, be added in an amount of 1/10 to 2 times that of the fat-soluble substance.

For the coating of the assembled hollow fiber membrane type blood purifier with a fat-soluble substance solution, there is a method that involves passing a coating solution obtained by dissolving 0.1 to 2.0% by mass of the fat-soluble substance in a 50 to 80% by mass aqueous solution of an alcohol, such as propanol, therethrough, creating a smooth coating thereof on the purifier for a predetermined time, e.g., 30 seconds to 60 minutes, preferably 40 seconds to 10 minutes, blowing off an excess of the coating solution with an air blow, and then removing the solvent by drying.

It is desirable that the coating temperature be 40°C or less, preferably 30°C or less, more preferably 25 °C or less. A coating temperature of more than 40°C probably increases the rate of the fat-soluble substance permeated into the membrane base material, having the possibility of not providing the above-described gentle albumin leakage.

<Step of Immobilizing Ketone and/or Alcohol Having 1 to 4 Carbons (Component A) on Hollow Fiber Membrane>

The ketone and/or the alcohol having 1 to 4 carbons can be contained in the hollow fiber membrane using any of the following methods. Examples thereof include a method that involves assembling a hollow fiber membrane type blood purifier, passing a fat-soluble vitamin solution therethrough, and then drying the resultant in an atmosphere of a vapor of the ketone and/or the alcohol having 1 to 4 carbons. Specifically, using the ketone and/or the alcohol having 1 to 4 carbons as a coating solvent, drying conditions can also be devised to retain the atmosphere of a vapor of the ketone and/or the alcohol having 1 to 4 carbons. The ketone and/or the alcohol having 1 to 4 carbons can also be artificially contained in a drying gas to be introduced to retain the vapor atmosphere to immobilize the ketone and/or the alcohol having 1 to 4 carbons.

<Sterilization Treatment Step>

It is preferable for sterilization treatment to be applied to the above hollow fiber membrane type blood purifier. The sterilization method may be any method such as a radiosterilization method or a steam sterilization method. The radiosterilization method is preferable because the hollow fiber membrane containing a large amount of the fat-soluble substance has the risk of producing hollow fiber breakage due to extreme heating. The radiosterilization method can use any of an electron beam, a gamma beam, an X-ray, and the like. The irradiation dose of a radiation is typically 5 to 50 kGy for a y beam or an electron beam; however, irradiation in the dose range of 20 to 40 kGy is preferable.

**Examples**

The present invention will be described below in further detail with reference to Examples.
Various measurement methods used in Examples will first be described.

<Quantification of Content of Fat-soluble Substance Present in Entire Hollow Fiber Membrane>

A hollow fiber membrane type blood purifier was disassembled to take a hollow fiber membrane, which was washed with water and then vacuum-dried at 40°C to a constant weight. After drying, 0.2 $m^2$ of the hollow fiber membrane was weighed out into a glass bottle, and 50 mL of N-methyl-2-pyrrolidone was added thereto, followed by stirring and dissolution.
Quantification was carried out by a liquid chromatography method, and the amount of a fat-soluble substance in the extract was determined using a calibration curve obtained from the peak area of a standard solution of the fat-soluble substance. Specifically, a high-performance liquid chromatographic system (UV-2075 plus intelligent UV/VIS Detector, PU-2080 plus intelligent HPLC pump, CO-2065 plus intelligent column oven, AS-2057 plus intelligent sampler, from JASCO Co., Ltd.,) was equipped with a column (Inertsil C8-3 $\mu$m (4.6 $\phi \times$ 250 mm) + ODP-50 6E (4.6 $\phi \times$ 250 mm)); N-methyl-2-pyrrolidone as a mobile phase was passed therethrough at a column temperature of 40°C and a flow rate of 0.5 mL/min; and the fat-soluble substance concentration was determined from the area under an absorption peak at a wavelength of 295 nm with a UV detector. The content (mg/$m^2$) of the fat-soluble substance present in the entire hollow fiber membrane was determined from the resultant concentration and the mass of the dissolved hollow fiber membrane.

<Quantification of Content of Fat-soluble Substance Present in Surface of Hollow Fiber Membrane>

The amount of the fat-soluble substance present in the hollow fiber membrane surface was measured as follows.
A hollow fiber membrane type blood purifier was disassembled to take a hollow fiber membrane, which was washed with water and then vacuum-dried at 40°C to constant weight. After drying, 0.2 $m^2$ of the hollow fiber membrane was weighed out into a glass bottle; 80 mL of a 1% by mass Triton X-100 (Kishida Chemical Do., Ltd., chemical grade) aqueous solution was added thereto; and the fat-soluble substance was extracted at room temperature for 60 minutes while applying ultrasonic vibration.
Quantification was carried out by a liquid chromatography method, and the amount of the fat-soluble substance in the extract was determined using the calibration curve obtained from the peak area of the standard solution of the fat-soluble substance. Specifically, the high-performance liquid chromatographic system (pump: JASCO PU-1580, detector: Shimadzu RID-6A, autoinjector: Shimadzu SIL-6B, data processing: Tosoh GPC-8020, and Column Oven: GL Sciences556) was equipped with the column (ODP-506E packed column for HPLC from Shodex Asahipak), through which methanol for high-performance liquid chromatography as a mobile phase was then passed at a column temperature of 40°C and a flow rate of 1 mL/min, followed by determining the fat-soluble substance concentration from the absorption peak area at a wavelength of 295 nm with a UV detector.

The content of the fat-soluble substance present in the hollow fiber membrane surface is the content (mg/$m^2$) of the fat-soluble substance extracted from the hollow fiber membrane surface. The fat-soluble substance partially oxidized by sterilization treatment was also included in the amount of the fat-soluble substance present in the hollow fiber membrane surface. Thus, to determine the amount of the partially oxidized fat-soluble substance, the fat-soluble substance to be used for preparing a calibration curve was exposed to 50 kGy of radiation in the air in advance; the absorption peak of the partially oxidized fat-soluble substance was determined in advance; and the peak was included in the peak group used for area calculating for addition.

The content (mg/$m^2$) of the fat-soluble substance present in the hollow fiber membrane surface is shown in the following Table 1. In Table 1, the mass of the fat-soluble substance contained in the hollow fiber membrane surface is denoted by "VE content".

<Proportion of Content of Fat-soluble Substance Present in Hollow Fiber Membrane Surface with respect to Content of Fat-soluble Substance Present in Entire Hollow Fiber Membrane>

The proportion of the content of the fat-soluble substance present in the hollow fiber membrane surface with respect to the content of the fat-soluble substance present in the entire hollow fiber membrane was calculated from the content of the fat-soluble substance present in the entire hollow fiber membrane and the content of the fat-soluble substance present in the hollow fiber membrane surface measured as described above. In Table 1, it is denoted by "proportion (%) of membrane surface VE".

<β2MG Clearance Rate>

Trisodium citrate dehydrate (30 g), dextrose (23.2 g), citric acid monohydrate (3.58 g), and sodium dihydrogenphosphate dehydrate (2.51 g) were dissolved in 1 L of water for injection to prepare an anticoagulant agent (CPD). CPD (614 mL) was added to fresh bovine blood (5 L) to provide an anticoagulated bovine blood. The resultant blood was centrifuged at 3500 rpm for 20 minutes to separate plasma. The resultant plasma was adjusted to a total protein concentration of 6.5 ± 0.5 g/dL by dilution with physiological saline. In addition, 1 mg/L of β2MG (Eiken Chemical Co., Ltd.) was added thereto to make a test fluid on the blood side. Separately, a test fluid on the dialysis side was prepared by dissolving 121.54 g of sodium chloride, 2.98 g of potassium chloride, 8.14 g of magnesium chloride, 9.83 g of sodium acetate, and 10 g of dextrose in 20 L of pure water. Both of the test fluid on the blood side and the test fluid on the dialysis side were maintained at 37 ± 1°C and subjected to testing.

A blood purifier having a membrane area of 1.5 $m^2$ was set in the "closed circuit" shown in Figure 2; the inlet flow rate on the blood side (QBi) of the blood purifier was adjusted to 200 mL/min, the flow rate on the blood outlet side (QBo), to 185 mL/min, and the filtration flow rate (QF), to 15 mL/min, and circulation was performed for 1 hour. The blood purifier was reset in "single circuit" shown in Figure 1 one hour later; the inlet flow rate on the blood side (QBi) of the blood purifier was adjusted to 200 mL/min; the flow rate on the blood outlet side (QBo), to 185 mL/min; and the dialysis fluid inlet flow rate (QDi), to 500 mL/min. The blood pump used was MARLOW-520S from Watson Co., Ltd., and the dialyzer used was DBG-03 from Nikkiso Ltd. Seven minutes after operation, fluids were sampled from the outlets of the blood supply fluid and the test fluid on the blood side and the outlet of the test fluid on the dialysis fluid side. The respective β2MG concentrations, CBi, CBo, and CDo, were quantified with a full-automatic analyzer, BM6050 (from JEOL Ltd.), using LZ test "Eiken" β2-M reagent (from Eiken Chemical Co., Ltd.). The clearance (CL) was calculated by the above expression (1).

<Albumin Leakage Amount>

The anticoagulated bovine blood was used and diluted with bovine plasma or physiological saline to adjust to a hematocrit of 35 ± 2% and a total protein concentration of 6.5 ± 0.5 g/dL. A commercially available dialysis fluid (Kindaly dialysis fluid AF2 from Fuso Pharmaceutical Industries, Ltd.) was fed through the blood flow channel of the blood purifier at 500 mL/min by one pass while circulating the blood through the blood flow channel of the blood purifier at 200 mL/min, and dialysis was performed while conducting ultrafiltration at 6 ml/min/$m^2$. To measure the 3 items of the total integrated value of albumin leakage amount (= the 4-hour integrated value of the albumin leakage amount), the proportion of the 5-minute integrated value of the albumin leakage amount with respect to the 1-hour integrated value of the albumin leakage amount, and the proportion of the 5-minute integrated value of the albumin leakage amount with respect to the 4-hour integrated value of the albumin leakage amount, the albumin leakage amount was measured under one condition 3 times, i.e., at the times of (1) the 5-minute integration, (2) the 1-hour integration, and (3) the 4-hour integration. DBG-03 from Nikkiso Ltd. was used for feeding the dialysis fluid. To prevent hemolysis, the blood purifier was sufficiently purged with physiological saline in advance. The feeding of the dialysis fluid and the ultrafiltration were started simultaneously with the start of bovine blood circulation, and the sampling of a ultrafiltrated fluid was started.

When the sampling of only the ultrafiltrated fluid is difficult, all dialysis waste fluid may be collected. The ultrafiltrated fluid or the dialysis waste fluid was sampled (1) for 0 to 5 minutes, (2) 5 minutes to 1 hour, and (3) 1 to 4 hours after the start of circulation to make a test fluid (1), a test fluid (2), and a test fluid (3). Each test fluid was well mixed, and the concentration of albumin (unit: g/mL) in the test fluid was quantified on an autoanalyzer, Biolis24i (Tokyo Boheki Medical System), using Iatro U-ALB (TIA) reagent (from Mitsubishi Chemical Medience Co., Ltd.). The total integrated value of albumin leakage amount (= the 4-hour integrated value of the albumin leakage amount), the proportion of the 5-minute integrated value of the albumin leakage amount with respect to the 1-hour integrated value of the albumin leakage amount, and the proportion of the 5-minute integrated value of the albumin leakage amount with respect to the 4-hour integrated value of the albumin leakage amount were calculated by the following expressions (11), (12), (13), (14), and (15) from the concentrations of the test fluids (1) to (3) and the ultrafiltrated fluid amounts (unit: mL).

[Expression 11]

(5-Minute integrated value of albumin leakage amount) = (test fluid (1) concentration) × (500 × 5 + 5-minute integrated ultrafiltrated amount) ・・・(11)

[Expression 12]

(1-Hour integrated value of albumin leakage amount) = (5-minute integrated value of albumin leakage amount) + (test fluid (2) concentration) × (500 × 55 + 55-minute integrated ultrafiltrated amount) ・・・(12)

[Expression 13]

(4-Hour integrated value of albumin leakage amount) = (1-hour integrated value of albumin leakage amount) + (test fluid (3) concentration) × (500 × 180 + 180-minute integrated ultrafiltrated amount) ・・・(13)

[Expression 14]

(Proportion of the 5-minute integrated value of the albumin leakage amount with respect to the 1-hour integrated value of the albumin leakage amount) = (5-minute integrated value of albumin leakage amount) ÷ (1-hour integrated value of albumin leakage amount) × 100 (%) ・・・(14)

[Expression 15]

$$\text{(Proportion of the 5-minute integrated value of the albumin leakage amount with respect to the 4-hour integrated value of the albumin leakage amount)} = \text{(5-minute integrated value of albumin leakage amount)} \div \text{(4-hour integrated value of albumin leakage amount)} \times 100\ (\%) \quad \cdots (15)$$

To quantify variations in the total albumin leakage amount, 10 modules were measured for the total leakage amount to calculate the CV value for measured values (= (standard deviation of measured values) ÷ (average of measured values)).

<Measurement of Content of Component A>

The content of a ketone and/or an alcohol having 1 to 4 carbons (hereinafter, referred to as "component A") per gram of the hollow fiber membrane in a hollow fiber membrane type blood purifier was measured by the following method.

[Case of Blood Purifier Filled with Water]

A blood purifier was disassembled; the hollow fiber membrane was separated; and water was removed therefrom by centrifuging the membrane at 3500 rpm for 10 minutes using a commercially available centrifuge. Of the hollow fiber membrane from which water was removed, 0.25 g was weighed out (this mass was called "w1 (g)"). After dissolution by adding 25 g of N-methyl-2-pyrrolidone, the component A, diethyl ether, and tetrahydrofuran were quantified by gas chromatography (GC). The concentration x (%) of the component A, diethyl ether, and tetrahydrofuran in the recovered solution was determined using a calibration curve obtained from the appropriate peak area of the component A.

Specifically, an autoinjector (AOC-20i/AOC-17) was connected to a gas chromatograph (GC-2014/GC14B from Shimadzu Corporation), and the above concentration was calculated from the absorption peak obtained with FID detector (hydrogen flame ionization detector). The content of the component A per gram of the hollow fiber membrane ("D (g/g of the hollow fiber membrane) ") was calculated by substituting the values w1 and x into the following expressions (8) to (9). In Table 1, the concentration is converted to "μg/g of the hollow fiber membrane" and is denoted by "mg/gHF".
[Expression 16]

$$\text{(Mass of component A in solution)} = x \times (w1 + w2) \div 100 \quad \cdots (8)$$

[Expression 17]

$$D = \text{(Mass of component A in solution)} \div w1 \quad \cdots (9)$$

[Case of Dry Blood Purifier]

A hollow fiber membrane type blood purifier was disassembled, and a hollow fiber membrane bundle was separated. The bundle was dried to constant weight in a desiccator using calcium chloride as a drying agent and weighed for mass ("w3 (g)"), and N-methyl-2-pyrrolidone ("w4 (g)") was added thereto, which was then stirred and dissolved, followed by calculating the concentration y (w/w%) of the component A by the above method. Subsequently, the content of the component A per gram of the hollow fiber membrane was calculated by substituting w3 and y into the following expression (16).
[Expression 18]

$$D = y \times (w3 + w4) \div w3 \div 100 \quad \cdots (10)$$

Example 1

Polysulfone (P-1700 from Solvay Co., Ltd.) (17.5% by weight) and polyvinylpyrrolidone (K90 from BASF Co., Ltd.) (3.5% by weight) were dissolved in N,N-dimethylacetamide (79.0% by weight) to make a uniform solution. Here, the mixing proportion of polyvinylpyrrolidone with respect to polysulfone in a membrane formation stock solution was 20.0% by weight. The membrane formation stock solution is maintained at 60°C, extruded together with the inner solution consisting of a mixed solution of 58.1% by weight N,N-dimethylacetamide and 41.9% by weight water through a double-wall annular spinning orifice, passed through a 0.96-m air gap, immersed in a coagulation bath consisting of water at 75°C, and rolled up at 80 m/minute. The rolled-up fiber bundle was cut and then washed by applying a hot water shower at 80°C from above of the cut surface of the bundle over 2 hours to remove the remaining solvent in the membrane, and the membrane was further dried to provide a dry membrane having a water content of less than 1%. In addition, the dry membrane was packed in a tubed container having 2 nozzles for introducing and delivering a liquid; both the ends of the container was embedded in a urethane resin; and then the hardened urethane portions were cut for working into the ends at which the hollow fiber membrane was opened. Both the ends were loaded with header caps having nozzles for introducing (delivering) blood for building-up into the form of a blood purifier apparatus having a hollow fiber membrane area of 1.5 $m^2$.

A coating solution in which 0.45% by weight of $\alpha$-tocopherol (special grade, from Wako Pure Chemical Industries Ltd.) as a fat-soluble substance was dissolved in a 57% by weight aqueous solution of isopropanol was passed through the lumen of the hollow fiber membrane for 52 seconds via the blood introduction nozzle of the blood purifier apparatus at a room temperature of 25°C to contact the lumen with the fat-soluble substance. In addition, after removing the residual solution in the lumen by air flushing, a dry air in an isopropanol atmosphere at 24°C, into which isopropanol vapor had been blown, was passed therethrough for 30 minutes to drying-remove the solvent to immobilize the fat-soluble substance by coating. A flow channel on the blood side and a flow channel on the filtrate side in the blood purifier apparatus were filled with an aqueous solution containing 0.06% sodium pyrosulfite as a sterile protective agent and further containing 0.03% sodium carbonate for adjusting pH, as a wetting step, followed by sterilization by irradiation with a $\gamma$-ray at 25 kGy in a state in which each nozzle was hermetically sealed to provide a blood purifier apparatus of the present invention.

As a result of measuring the content of the fat-soluble substance of the hollow fiber membrane in the resultant blood purifier, the content was 60.0 mg/$m^2$; the proportion of the fat-soluble substance present in the inner surface with respect to the fat-soluble substance present in the entire hollow fiber membrane was 70% by mass; the $\beta$2MG clearance was 70 mL/min; and the content of the component A in 1 g of the hollow fiber membrane was 2000 $\mu$g.

Example 2

A blood purifier was obtained in the same way as that in Example 1 except for setting the amount of N,N-dimethylacetamide in the inner solution at 57.1% by weight and the concentration of the fat-soluble substance in the coating solution at 0.15% by mass and blowing ethanol vapor into the dry air. As a result of measuring the content of the fat-soluble substance of the hollow fiber membrane in the resultant blood purifier, the content was 20.0 mg/$m^2$; the proportion of the fat-soluble substance present in the inner surface with respect to the fat-soluble substance present in the entire hollow fiber membrane was 70% by mass; the $\beta$2MG clearance was 70 mL/min; and the content of the component A (which was ethanol here) in 1 g of the hollow fiber membrane was 2000 $\mu$g.

Example 3

A blood purifier was obtained in the same way as that in Example 1 except for setting the amount of N,N-dimethylacetamide in the inner solution at 59.5% by weight and the concentration of the fat-soluble substance in the coating solution at 0.90% by mass and blowing acetone vapor into the dry air. As a result of measuring the content of the fat-soluble substance of the hollow fiber membrane in the resultant blood purifier, the content was 200.0 mg/$m^2$; the proportion of the fat-soluble substance present in the inner surface with respect to the fat-soluble substance present in the entire hollow fiber membrane was 70% by mass; the $\beta$2MG clearance was 70 mL/min; and the content of the component A (which was acetone here) in 1 g of the hollow fiber membrane was 2000 $\mu$g.

Example 4

A blood purifier was obtained in the same way as that in Example 1 except for setting the amount of N,N-dimethylacetamide in the inner solution at 55.0% by weight and using polyether sulfone represented by the chemical formula (2) (4800P from Sumitomo Chemical Co., Ltd.) in place of polysulfone represented by the chemical formula (1). As a result of measuring the content of the fat-soluble substance of the hollow fiber membrane in the resultant blood purifier, the content was 60.0 mg/$m^2$; the proportion of the fat-soluble substance present in the inner surface with respect

to the fat-soluble substance present in the entire hollow fiber membrane was 70% by mass; the β2MG clearance was 70 mL/min; and the content of the component A . (which was isopropanol here) in 1 g of the hollow fiber membrane was 2000 μg.

Example 5

A blood purifier was obtained in the same way as that in Example 1 except for setting the amount of N,N-dimethylacetamide in the inner solution at 55.5% by weight. As a result of measuring the content of the fat-soluble substance of the hollow fiber membrane in the resultant blood purifier, the content was 60.0 mg/$m^2$; the proportion of the fat-soluble substance present in the inner surface with respect to the fat-soluble substance present in the entire hollow fiber membrane was 70% by mass; the β2MG clearance was 65 mL/min; and the content of the component A (which was isopropanol here) in 1 g of the hollow fiber membrane was 2000 μg.

Example 6

A blood purifier was obtained in the same way as that in Example 1 except for passing the fat-soluble substance coating solution through the lumen of the hollow fiber membrane at a temperature of 40°C, followed by drying. As a result of measuring the content of the fat-soluble substance of the hollow fiber membrane in the resultant blood purifier, the content was 60.0 mg/$m^2$; the proportion of the fat-soluble substance present in the inner surface with respect to the fat-soluble substance present in the entire hollow fiber membrane was 45% by mass; the β2MG clearance was 70 mL/min; and the content of the component A (which was isopropanol here) in 1 g of the hollow fiber membrane was 10 μg.

Example 7

A blood purifier was obtained in the same way as that in Example 1 except for passing the fat-soluble substance coating solution through the lumen of the hollow fiber membrane at a temperature of 20°C, followed by drying. As a result of measuring the content of the fat-soluble substance of the hollow fiber membrane in the resultant blood purifier, the content was 60.0 mg/$m^2$; the proportion of the fat-soluble substance present in the inner surface with respect to the fat-soluble substance present in the entire hollow fiber membrane was 90% by mass; the β2MG clearance was 70 mL/min; and the content of the component A (which, was isopropanol here) in 1 g of the hollow fiber membrane was 3000 μg.

Comparative Example 1

A blood purifier was obtained in the same way as that in Example 1 except for setting the amount of N,N-dimethylacetamide in the inner solution at 55.5% by weight and not using the fat-soluble substance coating step and the component A immobilization step. As a result of measuring the content of the fat-soluble substance of the hollow fiber membrane in the resultant blood purifier, the content was 0 mg/$m^2$; the proportion of the fat-soluble substance present in the inner surface with respect to the fat-soluble substance present in the entire hollow fiber membrane was 0% by mass; the β2MG clearance was 70 mL/min; and the content of the component A in 1 g of the hollow fiber membrane was 0 μg.

Comparative Example 2

A blood purifier was obtained in the same way as that in Example 1 except for setting the amount of N,N-dimethylacetamide in the inner solution at 48.0% by weight. As a result of measuring the content of the fat-soluble substance of the hollow fiber membrane in the resultant blood purifier, the content was 60 mg/$m^2$; the proportion of the fat-soluble substance present in the inner surface with respect to the fat-soluble substance present in the entire hollow fiber membrane was 70% by mass; the β2MG clearance was 45 mL/min; and the content of the component A (which was isopropanol here) in 1 g of the hollow fiber membrane was 2000 μg.

Comparative Example 3

A blood purifier was obtained in the same way as that in Comparative Example 2 except for not using the fat-soluble substance coating step. As a result of measuring the content of the fat-soluble substance of the hollow fiber membrane in the resultant blood purifier, the content was 0 mg/$m^2$; the proportion of the fat-soluble substance present in the inner surface with respect to the fat-soluble substance present in the entire hollow fiber membrane was 0% by mass; the β2MG clearance was 45 mL/min; and the content of the component A in 1 g of the hollow fiber membrane

was 0 μg.

Comparative Example 4

A blood purifier was obtained in the same way as that in Example 1 except for dissolving 18.0% by weight of polyether sulfone and 3.0% by weight of polyvinylpyrrolidone in 74.5% by weight N,N-dimethylacetamide and 4.5% by weight of water to make a uniform solution, setting the amount of N,N-dimethylacetamide in the inner solution at 46.0% by weight, and using an 57% by weight aqueous solution of isopropanol not containing α-tocopherol as a coating solution used in the fat-soluble substance coating step. As a result of measuring the content of the fat-soluble substance of the hollow fiber membrane in the resultant blood purifier, the content was 0 mg/$m^2$; the proportion of the fat-soluble substance present in the inner surface with respect to the fat-soluble substance present in the entire hollow fiber membrane was 0% by mass; the β2MG clearance was 60 mL/min; and the content of the component A (which was isopropanol here) in 1 g of the hollow fiber membrane was 2000 μg.

Comparative Example 5

A blood purifier was obtained in the same way as that in Example 1 except for passing the fat-soluble substance coating solution through the lumen of the hollow fiber membrane at a temperature of 55°C, followed by drying. As a result of measuring the content of the fat-soluble substance of the hollow fiber membrane in the resultant blood purifier, the content was 60.0 mg/$m^2$; the proportion of the fat-soluble substance present in the inner surface with respect to n the fat-soluble substance present in the entire hollow fiber membrane was 30% by mass; the β2MG clearance was 70 mL/min; and the content of the component A (which was isopropanol here) in 1 g of the hollow fiber membrane was 2000 μg.

Comparative Example 6

A blood purifier was obtained in the same way as that in Example 1 except for removing the residual coating solution present in the lumen and then setting the drying temperature at 80°C without blowing component A vapor into the dry air. As a result of measuring the content of the fat-soluble substance of the hollow fiber membrane in the resultant blood purifier, the content was 60.0 mg/$m^2$; the proportion of the fat-soluble substance present in the inner surface with respect to the fat-soluble substance present in the entire hollow fiber membrane was 70% by mass; the β2MG clearance was 70 mL/min; and the content of the component A (which was isopropanol here) in 1 g of the hollow fiber membrane was 1 μg.

Comparative Example 7

A blood purifier was obtained in the same way as that in Example 1 except for removing the residual coating solution present in the lumen and then using a diethyl ether atmosphere as a dry air. Physical properties of the hollow fiber membrane in the resultant blood purifier was attempted to be measured, but could not be measured since the amount of fluid permeation in the membrane was insufficient.

Comparative Example 8

A blood purifier was obtained in the same way as that in Example 1 except for removing the residual coating solution present in the lumen and then using a tetrahydrofuran atmosphere as a dry air. Physical properties of the hollow fiber membrane in the resultant blood purifier was attempted to be measured, but could not be measured since the amount of fluid permeation in the membrane was insufficient.

For the blood purifiers of the above-described Examples and Comparative Examples, the albumin leakage amount over the entire period of 4-hour blood purification treatment, and the proportion of the albumin leakage amount for 5 minutes after the start of blood purification treatment (B) with respect to the albumin leakage amount from the start of blood purification treatment (A) until 1 hour thereafter (5-minute leakage proportion (B/A)) are shown in Table 1.

[Table 1]

| | VE CONTENT [mg/m$^2$] | Proportion of Membrane Surface VE [%] | β 2MG CL [mL/min] | Content of Component A [mg/gHF] | Albumin Leakage | | |
|---|---|---|---|---|---|---|---|
| | | | | | 5-Minute Leakage Proportion (B/A) [%] | Total Leakage Amount [g/ session] | Variation in Total Leakage Amount [%] |
| Example 1 | 60 | 70 | 70 | 2000 | 36 | 1.5 | 28 |
| Example 2 | 20 | 70 | 70 | 2000 | 39 | 1.7 | 18 |
| Example 3 | 200 | 70 | 70 | 2000 | 35 | 1.6 | 31 |
| Example 4 | 60 | 70 | 70 | 2000 | 35 | 1.4 | 34 |
| Example 5 | 60 | 70 | 65 | 2000 | 42 | 1.0 | 24 |
| Example 6 | 60 | 45 | 70 | 10 | 39 | 1.8 | 45 |
| Example 7 | 60 | 90 | 70 | 3000 | 33 | 1.2 | 28 |
| Comparative Example 1 | 0 | 0 | 70 | 0 | 69 | 2.2 | 25 |
| Comparative Example 2 | 60 | 70 | 45 | 2000 | 62 | 0.7 | 32 |
| Comparative Example 3 | 0 | 0 | 45 | 0 | 64 | 0.8 | 25 |
| Comparative Example 4 | 0 | 0 | 60 | 2000 | 63 | 2.0 | 28 |
| Comparative Example 5 | 60 | 30 | 70 | 2000 | 55 | 2.0 | 36 |
| Comparative Example 6 | 55 | 60 | 65 | 1 | 40 | 1.0 | 97 |

VE Content: Content of Fat-soluble Substance in Hollow Fiber Membrane Surface
Proportion of Membrane Surface VE: Mass Proportion of Fat-soluble Substance Present in Hollow Fiber Membrane Surface with respect to Fat-soluble Substance Present in Entire Hollow Fiber Membrane
β2MG CL: β 2MG Clearance for Blood Purifier
Total Leakage Amount:. Albumin Leakage Amount over Total Period (4 Hours) of Blood treatment
Content of Component A: Mass of Component A Contained in 1 g of Hollow Fiber Membrane
5-Minute Leakage Proportion (B/A): Albumin Leakage Amount for 5 Minutes after Start of Blood Purification Treatment (B)/Albumin Leakage Amount from Start of Blood Purification Treatment until 1 Hour thereafter(A)
Variation in Total Leakage Amount: CV Value in Measurement of Total Albumin Leakage Amount (n = 10)

Comparison between Example 1 and Comparative Example 1 showed that the fat-soluble substance contained in the hollow fiber membrane reduced albumin leakage over the total period and further was effective in reducing the proportion of albumin leakage from the start of blood purification treatment until 5 minutes thereafter.

Comparison between Example 1 and Comparative Example 5 showed that the mass proportion of the fat-soluble substance present in the hollow fiber membrane surface with respect to the fat-soluble substance present in the entire hollow fiber membrane affected a reduction in the total albumin leakage amount and a reduction in the 5-minute leakage ratio.

Comparison between Examples 1 and 5 and Comparative Examples 1, 2, and 3 showed that the β2MG clearance rate in the range of 65 mL/min or more affected a reduction in the total albumin leakage amount due to the fat-soluble substance contained and a reduction in the 5-minute leakage ratio.

In addition, Comparison between Examples 1 and 4 showed that polysulfone and polyether sulfone as a material of the hollow fiber membrane also exhibited a similar effect.

## Industrial Applicability

The hollow fiber membrane for blood treatment according to the present invention has high solute permeability enabling a certain amount of albumin leakage to be achieved in blood treatment in addition to having improved fractionating properties between β2MG and albumin, and further has a reduced amount of albumin leakage at the beginning of the blood treatment, i.e., continues to gently leak albumin over the entire period of the blood treatment, to enable the provision of a blood purifier capable of simultaneously reducing dialysis amyloidosis and dialysis disequilibrium syndrome as dialysis complications. The hollow fiber membrane for blood treatment according to the present invention is industrially applicable in the form of a blood purifier, such as a hemodialyzer, a hemofilter, or a hemodiafilter.

## Claims

1. A polysulfone hollow fiber membrane for blood treatment, comprising a polysulfone resin, a hydrophilic polymer, and a fat-soluble vitamin, wherein:

   the fat-soluble vitamin is selected from the group consisting of vitamin A, vitamin D, vitamin E, vitamin K and ubiquinone;
   the amount of the fat-soluble vitamin present in the surface of the membrane is 10 to 300 mg per square meter;
   the content of the fat-soluble vitamin present in the surface of the membrane is 40 to 95% by mass when the content of the fat-soluble vitamin present in the entire membrane is assumed as 100% by mass;
   the clearance rate of β2 microglobulin (β2MG) converted to that for 1.5 $m^2$ for a blood purifier assembled using the membrane is 65 mL/min or more **characterized in that** the hollow fiber membrane contains 2 to 3000 μg of a ketone and/or an alcohol having 1 to 4 carbons per gram of the hollow fiber membrane, and
   wherein the content of the fat-soluble vitamin in the hollow fiber membrane surface and in the entire membrane as well as the clearance-rate of β2MG are measured by the method specified in the description.

2. The polysulfone hollow fiber membrane for blood treatment according to claim 1, wherein the albumin leakage amount from the start of blood treatment until 5 minutes thereafter, determined as described in the description, is 50% by mass or less when the total albumin leakage amount from the start of blood treatment until 1 hour thereafter is assumed as 100% by mass.

3. The polysulfone hollow fiber membrane for blood treatment according to claim 1 or 2, wherein the total albumin leakage amount over the entire period of the blood treatment, determined as described in the description, is 1.0 g/session or more.

4. The polysulfone hollow fiber membrane for blood treatment according to any one of claims 1 to 3, wherein the clearance of β2MG converted to that for 1.5 $m^2$, determined as described in the description, is 70 mL/min or more.

## Patentansprüche

1. Polysulfon-Hohlfasermembran für die Blutbehandlung, umfassend ein Polysulfonharz, ein hydrophiles Polymer und ein fettlösliches Vitamin, wobei:

   das fettlösliche Vitamin aus der Gruppe ausgewählt ist, die aus Vitamin A, Vitamin D, Vitamin E, Vitamin K und Ubichinon besteht;
   die Menge des fettlöslichen Vitamins, das in der Oberfläche der Membran vorhanden ist, 10 bis 300 mg pro $m^2$ beträgt;
   der Gehalt an fettlöslichem Vitamin, das in der Oberfläche der Membran vorhanden ist, 40 bis 95 Massen-% beträgt, wenn der Gehalt an fettlöslichem Vitamin, das in der gesamten Membran vorhanden ist, als 100 Massen-% angenommen wird;
   die Clearance von β2-Mikroglobulin (β2MG), die auf 1,5 $m^2$ für einen mit Hilfe der Membran zusammengebauten Blutreiniger umgerechnet ist, 65 ml/min oder mehr beträgt, **dadurch gekennzeichnet, dass** die Hohlfasermembran 2 bis 3000 μg eines Ketons und/oder eines Alkohols, der 1 bis 4 Kohlenstoffatome aufweist, pro Gramm der Hohlfasermembran enthält,
   wobei der Gehalt an dem fettlöslichen Vitamin in der Oberfläche der Hohlfasermembran und in der gesamten Membran sowie die Clearance von β 2MG nach dem in der Beschreibung näher beschriebenen Verfahren

gemessen werden.

2. Polysulfon-Hohlfasermembran für die Blutbehandlung gemäß Anspruch 1, wobei die Albuminaustrittsmenge vom Beginn der Blutbehandlung bis 5 Minuten danach, die so bestimmt wird, wie es in der Beschreibung beschrieben ist, 50 Massen-% oder weniger beträgt, wenn die Gesamtalbuminaustrittsmenge vom Beginn der Blutbehandlung bis 1 Stunde danach als 100 Massen-% angenommen wird.

3. Polysulfon-Hohlfasermembran für die Blutbehandlung gemäß Anspruch 1 oder 2, wobei die Gesamtalbuminaustrittsmenge über die gesamte Dauer der Blutbehandlung, die so bestimmt wird, wie es in der Beschreibung beschrieben ist, 1,0 g/Sitzung oder mehr beträgt.

4. Polysulfon-Hohlfasermembran für die Blutbehandlung gemäß einem der Ansprüche 1 bis 3, wobei die Clearance von β2MG, umgerechnet auf 1,5 $m^2$, die so bestimmt wird, wie es in der Beschreibung beschrieben ist, 70 ml/min oder mehr beträgt.

## Revendications

1. Membrane à fibres creuses en polysulfone pour le traitement du sang, comprenant une résine de polysulfone, un polymère hydrophile et une vitamine liposoluble, dans laquelle:

   la vitamine liposoluble est choisie dans le groupe consistant en la vitamine A, la vitamine D, la vitamine E, la vitamine K et l'ubiquinone;
   la quantité de vitamine liposoluble présente dans la surface de la membrane est 10 à 300 mg par mètre carré;
   la teneur en vitamine liposoluble présente dans la surface de la membrane est 40 à 95 % en masse lorsque la teneur en vitamine liposoluble présente dans toute la membrane est supposée être 100 % en masse;
   le taux de clairance de β2 microglobuline (β2MG) converti en celui pour 1,5 $m^2$ pour un purificateur de sang assemblé à l'aide de la membrane est 65 mL/min ou plus **caractérisée en ce que** la membrane à fibres creuses contient 2 à 3000 μg d'une cétone et/ou d'un alcool ayant 1 à 4 carbones par gramme de la membrane à fibres creuses, et
   dans laquelle la teneur en vitamine liposoluble dans la surface de la membrane à fibres creuses et dans toute la membrane ainsi que le taux de clairance de β 2MG sont mesurés par le procédé spécifié dans la description.

2. Membrane à fibres creuses en polysulfone pour le traitement du sang selon la revendication 1, dans laquelle la quantité de fuite d'albumine depuis le début du traitement du sang jusqu'à 5 minutes après, déterminée comme décrit dans la description, est 50 % en masse ou moins lorsque la quantité de fuite d'albumine totale depuis le début du traitement du sang jusqu'à 1 heure après est supposée être 100 % en masse.

3. Membrane à fibres creuses en polysulfone pour le traitement du sang selon la revendication 1 ou 2, dans laquelle la quantité de fuite d'albumine totale sur toute la période du traitement du sang, déterminée comme décrit dans la description, est 1,0 g/séance ou plus.

4. Membrane à fibres creuses en polysulfone pour le traitement du sang selon l'une quelconque des revendications 1 à 3, dans laquelle la clairance de β 2MG convertie en celle pour 1,5 $m^2$, déterminée comme décrit dans la description, est 70 mL/min ou plus.

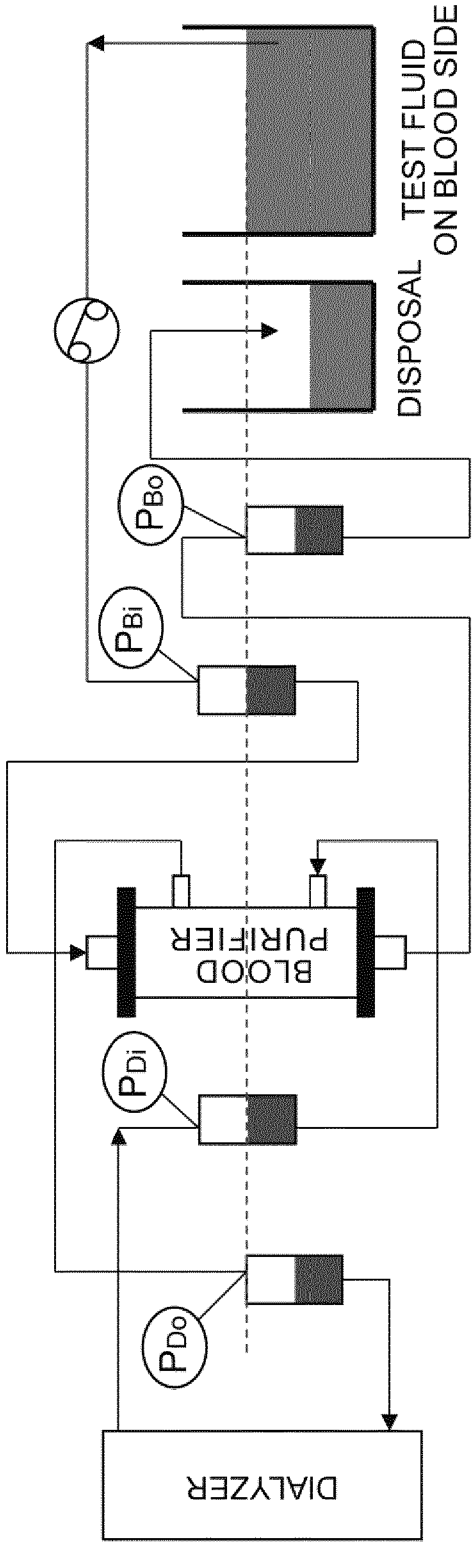
TEST FLUID
ON BLOOD SIDE
DISPOSAL
PBo
PBi
BLOOD
PURIFIER
PDi
PDo
DIALYZER

Fig.1

*Fig.2*

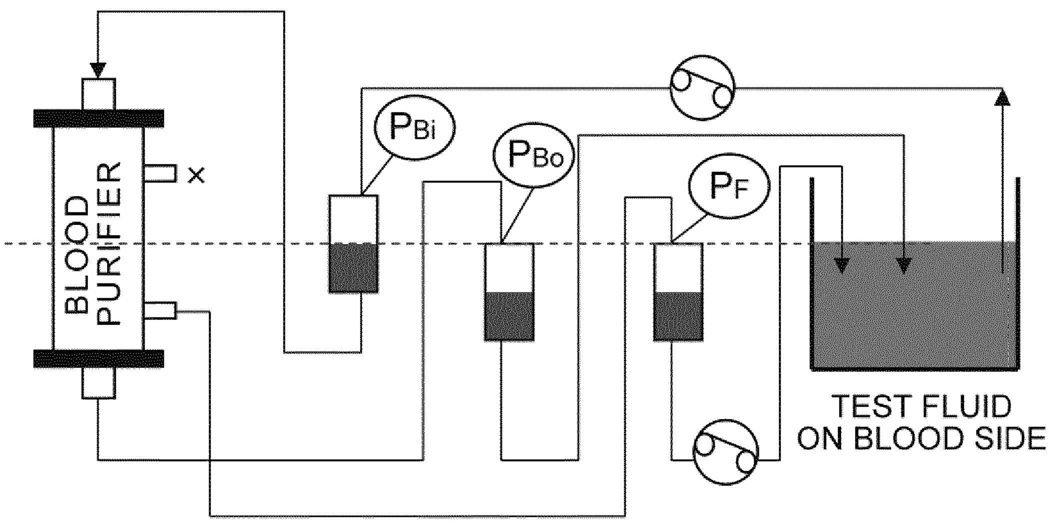

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 7178166 A
- JP 2006296931 A
- JP 2011212638 A
- JP 2005270631 A
- EP 0923955 A
- JP 4038583 B
- WO 9852683 A


### Non-patent literature cited in the description


- **TAKESHI SATOH.** *Journal of the Japanese Society for Dialysis Therapy,* 1996, vol. 29 (8), 1231-1245
- **MICHIO MINESHIMA.** Ketsueki Johkaki - Seinohhy-ohkano Kiso (Blood Purifier - Basis for Performance Evaluation). Nippon Medical Center, 2002